Europäisches Patentamt

European Patent Office

Office européen des brevets

(11) Publication number: **0 166 601**
A2

(12) **EUROPEAN PATENT APPLICATION**

(21) Application number: **85304518.5**

(22) Date of filing: **25.06.85**

(51) Int. Cl.⁴: **A 23 C 9/123**, A 23 G 9/00

(30) Priority: **26.06.84 GB 8416212**

(43) Date of publication of application: **02.01.86**
Bulletin **86/1**

(84) Designated Contracting States: **BE CH DE FR GB IT LI NL SE**

(71) Applicant: **JOHN & E STURGE LIMITED, Denison Road, Selby North Yorkshire YO8 8EF (GB)**

(72) Inventor: **Frost, Geoffrey Michael, 21 Spring Walk Brayton Selby, North Yorkshire, Y08 9DS (GB)**
Inventor: **Gregory, Keith William, 9 Garth View Hambleton Selby, North Yorkshire, Y08 9PS (GB)**

(74) Representative: **Watkins, Arnold Jack et al, European Patent Attorney Frank B. Dehn & Co. Imperial House 15-19 Kingsway, London WC2B 6UZ (GB)**

(54) **Lactase-containing compositions and their preparation.**

(57) A lactase enzyme-containing composition for use in the hydrolysis of lactose in foodstuffs comprises a mixture of a lactase, an emulsifying agent and, optionally, a stabilising agent, the composition being in particulate form with the lactase and, where present, the stabilising agent dispersed in the emulsifying agent. Processes for making such compositions are described as are milk based foodstuffs having reduced lactose content obtained by the incorporation therein of such compositions.

ACTORUM AG

- 1 -

12G 148-596

<u>Lactase-containing compositions and
their preparation</u>

This invention relates to lactase-containing
compositions and more particularly to such compositions
for use in the production of foodstuffs.

Lactases are enzymes used for the hydrolysis
of lactose to form glucose and galactose.  Lactases
are generally derived either from yeasts or moulds.
Although mould lactases are more stable, it is
in general preferred to use lactases derived from
yeasts which lactases may be obtained in high yields.
The present invention is concerned particularly
with compositions containing the less stable yeast
lactases, and thus the expression 'lactase' as
used herein is to be interpreted as referring specifically to yeast lactase.

It has been proposed, e.g. in GB-A-2063049,
DE-A-3040702 and GB-A-2076275, to include lactases
as an ingredient of ice cream, especially soft
ice cream.  These specifications describe various
ice cream recipes which include as ingredients
animal (e.g. butter) or vegetable fats, sucrose,
lactase preparation and emulsifying/stabilizing
combination.  Further ingredients referred to
are skimmed milk powder, demineralized whey powder
and dextrose (in GB-A-2063049); skimmed milk powder
and dextrose (in DE-A-3040702) and skimmed milk
powder and lactose (in GB-A-2076275).  All these
recipes involve inclusion in the formulation of a
substantial amount of lactose, this being an ingredient
of skimmed milk powder and demineralized whey powder.
The lactase converts part or all of the lactose present
into the sweeter and more soluble monosaccharides,
glucose and galactose.  In the absence of added
lactase, the amount of lactose which can be used in the formulation of ice cream is limited by the possibility of lactose crystals forming which cause the ice cream to have an undesirable 'sandy' texture. The primary purpose of using lactase in ice creams is to allow larger amounts of components of relatively high lactose content such as whey powder to be used in the ice cream formulation. The increased sweetness produced by hydrolysis of part or all of the lactose provides a secondary advantage and may allow a saving in the amount of sucrose added.

Lactases may also be added to other lactose containing foodstuffs, e.g. whey, milk, skim milk, milk-based drinks, etc. to convert some or all of the lactose to glucose and galactose. Such lactose hydrolysed foodstuffs are then suitable for consumption by lactose intolerant populations who are unable to digest lactose and so find normal milk and milk based products unacceptable.

When reconstituting milk powder, it is preferable to use added emulsifying/stabilizing agents to stabilize the casein micelles and the fat emulsion.

Thus a manufacturer producing reduced lactose or lactose-free products from lactose-containing starting materials generally must obtain separate supplies of lactase and of an emulsifying/stabilizing mixture and accurately control the amount of each included in the foodstuffs formulation so as to obtain the desired degree of lactose conversion.

It is an object of the present invention to provide a more convenient method whereby lactase enzyme may be incorporated into lactose-containing compositions, such as ice cream formulations or reconstituted milks.

In accordance with one aspect of the present invention, there is provided a lactase enzyme-containing composition for use in the hydrolysis of lactose in foodstuffs which comprises a mixture of a lactase, an emulsifying agent and, optionally, a stabilising agent, the composition being in particulate form with the lactase and, where present, the stabilising agent dispersed in the emulsifying agent.

It will be appreciated that the emulsifying agent, lactase and, where present, the stabilising agent will be incorporated in amounts which in use serve to provide the desired emulsification/stabilisation and conversion of lactose to sweet monosaccharides in the foodstuffs.

Known emulsifying/stabilising combinations may, if desired be employed in the compositions according to the invention. Such combinations include, for example those described in the above-mentioned specifications, two such combinations being available from Kirk Food A/S under the trade names 'PANISOL' XO SUPER A and 'PANISOL' Pl SUPER A. Fatty acid ester emulsifying agents and natural gum stabilisers are thus conveniently used, examples being the mixtures of mono- and diglycerides of fatty acids - mainly stearic and palmitic acids - and the guar gum and carrageenan which are present in 'PANISOL' XO SUPER A and 'PANISOL' Pl SUPER A. Other natural gums which may if desired be used are for example gum arabic and xanthan gum. The emulsifying agent of 'PANISOL' XO SUPER A is available separately as 'PANISOL' MO 48.

Emulsifying agents of the kind referred to above are solid at ambient temperatures. Thus, in order to produce emulsifying agent/lactase mixtures in which the lactase and, optionally, a stabilising agent are dispersed in the emulsifying agent, it is necessary to heat the emulsifying agent to an elevated temperature at which it is liquid, to incorporate the stabilising agent and the lactase at such elevated temperature and then to cool. With emulsifying agents consisting of mixtures of mono-and diglycerides of fatty acids such as stearic and palmitic acids, heating to temperatures of 70 to 80°C or thereabouts is preferred in order to obtain a sufficiently non-viscous liquid for ready mixing with the stabilising agent and the lactase. Because lactases are known to be heat sensitive materials, the use of such elevated temperatures could be expected to deactivate the enzyme.

We have discovered that compositions according to the invention which are in the form of dispersions of the lactase and optionally a stabilising agent in the emulsifying agent can be prepared without deactivation of the lactase.

Thus, according to a further aspect of the present invention, there is provided a process for preparing a composition according to the invention as hereinbefore defined which comprises heating an emulsifying agent (preferably an emulsifying agent which has a melting point not higher than 80°C,) to effect melting, dispersing the lactase and, if required, the stabilising agent in solid particulate form in the liquid emulsifying agent, cooling the mixture thereby obtained before deactivation of the lactase to an undesired extent has taken place and subdividing the composition during and/or after cooling thereby to obtain a product in particulate form. Provided the lactase used is in solid particulate form, which is conveniently in the form of a powder, it has surprisingly been found that any deactivation which takes place can be kept within acceptable limits.

An alternative process for preparing compositions according to the invention, which alternative process constitutes a further feature of the present invention, comprises heating an emulsifying/stabilising combination (preferably one which has a melting point not higher than 80°C, e.g. an emulsifying/stabilising combination as described in GB-A-2063049, DE-A-3040702 or GB-A-2076275 such as for example 'PANISOL' P1 SUPER A, or the similar emulsifying/stabilising combination 'PANISOL' XO SUPER A) to effect melting, dispersing the lactase in solid, particulate form in the liquid emulsifying/stabilising combination, cooling the mixture thereby obtained before deactivation of the lactase to an undesired extent has taken place and subdividing the composition during and/or after cooling thereby to obtain a product in particulate form.

The temperature at which incorporation of the lactase is effected will depend upon the emulsifying agent and will advantageously be as low as possible for the emulsifying agent used. Especially preferable emulsifying agents are those which melt at temperatures of no higher than about 55 to 70°C. With emulsifying agents consisting of mixtures of mono- and diglycerides of fatty acids such as stearic and palmitic acids, the use of temperatures below 80°C down to 70°C or even lower will reduce any tendency for deactivation of the enzyme. The temperature used should in general be the lowest that will allow adequate dispersion of the lactase (and the stabilising agent when also incorporated as part of the process of the invention).

The compositions according to the invention are in particulate form, conveniently in the form of granules. If desired, after mixing, the composition can be cooled and thereafter broken up into granules of a desired particle size. Alternatively, a cooling method such as for example spray cooling which provides a particulate product may be used. As stated above, the cooling must be effected before deactivation of the lactase to an undesired extent. In practice it may be necessary when mixing and cooling large quantities of the ingredients that the lactase enzyme should be at the elevated temperatures used for a substantial period of time. As can be seen from the examples below we have established that undesired deactivation of the lactase can be avoided even when it is subjected to elevated temperatures for periods of time of as long as two hours.

The preferred relative proportions of the emulsifying and stabilising agents in the compositions according to the invention will vary according to the particular emulsifying and stabilising agents used and the nature of the foodstuffs to which the compositions are to be added. In general the ratio by weight of emulsifying agent to stabilising agent will conveniently be in the range of 4:1 to 1:1, the amount of the emulsifying agent advantageously being not substantially greater than that required to emulsify the stabilising agent in the foodstuffs formulation. The preferred amount of lactase will similarly vary according to the nature of the foodstuffs to which the compositions are to be added, but will in general preferably be from 0.2g (5,000 EU) to 20g (500,000 EU) per 100g of the composition according to the invention. One EU is the quantity of lactase which hydrolyses one micromole of lactose in one minute at 30°C in a 5% w/v solution of lactose buffered at pH 6.2 with potassium phosphate and containing a 10 millimolar concentration of magnesium ions.

Following cooling, the compositions according to the invention can be stored at ambient temperatures or more preferably at lower temperatures (e.g. 4°C) at which the lactase enzyme has improved stability.

According to another aspect, the invention provides milk based foodstuffs having reduced lactose content obtained by the incorporation within said foodstuffs of the composition of the invention. Such foodstuffs may include directly consumable products such as ice cream and also fresh whole milk, skim milk and sweet whey; concentrates and spraydried products of the latter products; and reconstituted products of such concentrates and spraydried products.

The foodstuffs of the invention can be prepared in a conventional manner by mixing the composition of the invention with the remaining ingredients and by maintaining the mixture so obtained at a temperature and for a time sufficient to ensure the desired degree

- 7 -

of lactose hydrolysis has taken place. Thus, the composition may be incorporated into the foodstuff at a temperature generally of from 0 to 50°C and the mixture obtained then held at a temperature conveniently of from 35 to 45°C e.g. for 2 to 4 hours or more depending on the amount of lactose hydrolysis required. If desired the product may then be pasteurized or sterilised. Maintaining the mixture at a higher temperature such as 35 to 45°C will of course accelerate lactose hydrolysis but this must, in certain cases, be balanced against possible increased microbial contamination and it may sometimes be desirable for the mixture to be held at a lower temperature for a longer period to effect the necessary lactose hydrolysis. For example skim milk powder reconstituted by being dissolved in water with the composition of the invention may be suitable for consumption by a totally lactose intolerant population after about 24 hours at 4°C.

The following non-limiting Examples are provided to illustrate the invention:

EXAMPLE 1

20g of an emulsifier/stabiliser combination ('PANISOL' XO SUPER A) was heated to 80°C on a water bath and 0.4g of a lactase enzyme in powder form ('HYDROLACT' S 250 of John & E. Sturge Limited) was added with stirring. The mixture thus obtained was maintained at 80°C with stirring for a period of 30 minutes with samples being removed and rapidly cooled at intervals. The enzyme activity of each sample was then estimated as follows:

- 8 -

| Time (in mins) at 80°C before rapid cooling | Enzyme activity (in EU/g) |
|---|---|
| 0 | 400 |
| 5 | 400 |
| 10 | 430 |
| 15 | 410 |
| 20 | 360 |
| 30 | 430 |

The variations in enzyme activity were within the experimental error of the estimation, and the results show that the lactase enzyme was able to survive a temperature of 80°C for at least 30 minutes without significant loss of activity.

EXAMPLE 2

40g of 'PANISOL' MO 48 (the emulsifier of 'PANISOL' XO SUPER A)were heated on a water bath to 70°C and 20g of carrageenan (Sigma, Type 1) and 1.2 g of a lactase enzyme in powder form ('HYDROLACT' S 250) were added. The mixture thus obtained was maintained at 70°C with stirring for a period of 2 hours with samples being removed and rapidly cooled at intervals. The enzyme activity of each sample was then estimated as follows:

| Time (in mins) at 70°C before rapid cooling | Enzyme activity (in EU/g) |
|---|---|
| 0 | 460 |
| 15 | 430 |
| 30 | 400 |
| 45 | 380 |
| 60 | 400 |
| 90 | 360 |
| 120 | 310 |

These results show that, whilst there is some loss in enzyme activity over a period of two hours at 70°C, such loss is well within acceptable limits.

- 9 -

EXAMPLE 3

The procedure of Example 2 was repeated with the difference that the mixture was maintained at 80°C instead of 70°C for the 2 hour period. The enzyme activities of the samples were estimated as follows:

| Time (in mins) at 80°C before rapid cooling | Enzyme activity (in EU/g) |
|---|---|
| 0 | 390 |
| 10 | 340 |
| 20 | 280 |
| 30 | 270 |
| 45 | 260 |
| 60 | 230 |
| 90 | 200 |
| 120 | 200 |

Whilst these results show a greater loss in enzyme activity than that observed in Example 2 at 70°C, nonetheless such loss is still within acceptable limits.

The cooled products of Examples 1 to 3 can be broken up or spray cooled into granules of desired particle size for formulation into lactose-containing foodstuffs ingredients.

EXAMPLE 4

100 g of an emulsifier/stabilizer combination ('PANISOL' XO SUPER A) was heated to 70°C on a water bath and 4 g of a lactase enzyme in powder form ('HYDROLACT' S 250) was added with stirring. The mixture thus obtained was immediately rapidly cooled and the solid product obtained was granulated. The enzyme activity of the granulate was 600 EU/g.

EXAMPLE 5

100 g of an emulsifier/stabilizer combination ('PANISOL' Pl SUPER A) was heated to 70°C on a water bath and 4 g of a lactase enzyme in powder form ('HYDROLACT S 250) was added with stirring. The mixture thus obtained was immediately rapidly cooled and the solid product obtained was granulated. The enzyme activity of the granulate was 960 EU/g.

EXAMPLE 6

Ice cream was prepared in a conventional manner, e.g. as described in GB-A-2076275, using the following ingredients:

| | | |
|---|---|---|
| Sucrose | 12% | by weight |
| Dextrose | 3% | " " |
| Fat | 3.5% | " " |
| Skim milk powder | 5% | " " |
| Demineralized whey powder | 10% | " " |
| Granulate product of Example 4 | 0.65% | " " |
| Water                    ad | 100% | " " |

The ingredient mixture was heated to 40°C and maintained at that temperature for two hours whereafter, to prevent further enzyme action, it was pasteurized at 80°C. After holding for several hours at 2 to 10°C the pasteurized mixture was frozen in an ice-cream freezer and then hardened at -20 to -30°C.

The lactose content of the ice cream mixture was reduced to 4.2% from the initial content of 10% and the ice cream on removal from the freezer was found to be readily scoopable and to have good taste and texture.

EXAMPLE 7

Example 6 was as repeated using 0.4% by weight of the granulate product of Example 5 in place of the product of Example 4. The lactose content of the ice cream mixture was reduced to 4.9% from the initial content of 10% and the ice cream on removal from the freezer was found to be readily scoopable and to have good taste and texture.

EXAMPLE 8

Reconstituted milk was prepared by dissolving skim milk powder and the granulate product of Example 4 in water in the following quantities:

| | | |
|---|---|---|
| Skim milk powder | 9% | by weight |
| Granulate product of Example 4 | 0.5% " | " |
| Water                    ad | 100% " | " |

Samples of the reconstituted milk were incubated at 10°C, 25°C and 40°C. At 10°C 60% lactose hydrolysis was achieved in 16 hours; at 25°C 50% hydrolysis was achieved in 4 hours; and at 40°C 60% hydrolysis was achieved in 4 hours.

EXAMPLE 9

Example 8 was repeated substituting the granulate product of Example 5 for that of Example 4.

At 10°C 60% lactose hydrolysis was achieved in 16 hours; at 20°C 68% hydrolysis was achieved in 4 hours; and at 40°C 80% hydrolysis was achieved in 4 hours.

0166601

- 12 -

Claims:

1.    A lactase enzyme-containing composition for use in the hydrolysis of lactose in foodstuffs which comprises a mixture of a lactase, an emulsifying agent and, optionally, a stabilising agent, the composition being in particulate form with the lactase and, where present, the stabilising agent dispersed in the emulsifying agent.

2.    A composition according to claim 1 wherein the emulsifying agent comprises one or more fatty acid esters.

3.    A composition according to either of the preceding claims wherein the stabilising agent, where present, comprises a natural gum.

4.    A composition according to any preceding claim wherein either the emulsifying agent alone (a stabilising agent being optionally present) or the combination of the emulsifying agent and the stabilising agent (where present) has a melting point no higher than 80°C.

5.    A composition according to any preceding claim in the form of granules.

6.    A composition according to any preceding claim wherein the ratio by weight of emulsifying agent to stabilising agent (where present) is in the range of 4:1 to 1:1.

7.    A composition according to any preceding claim containing from 0.2 (5,000 EU) to 20 g (500,000 EU) of lactase per 100 g of the composition.

8.    A process for the preparation of a composition according to claim 1 which comprises

      either heating the emulsifying agent to effect melting and dispersing the lactase and, if required, the stabilising agent in solid particulate form in the liquid emulsifying agent,

      or heating a combination of the emulsifying agent and the stabilising agent to effect melting and dispersing the lactase in solid, particulate

- 13 -

form in the liquid combination,

the mixture thereby obtained in each case being subsequently cooled before deactivation of the lactase to an undesired extent has taken place, the composition being subdivided during and/or after cooling thereby to obtain a product in particulate form.

9. Milk based foodstuffs having reduced lactose content obtained by the incorporation therein of a composition according to any one of claims 1 to 7.

10. Foodstuffs according to claim 9 in the form of ice cream, fresh whole milk, skimmilk or sweet whey; or in the form of concentrates or spraydried products of the aforesaid products; or in the form of reconstituted products of such concentrates or spraydried products.

11. A process for the preparation of a foodstuff according to claim 9 wherein a composition according to any one of claims 1 to 7 is mixed with the remaining ingredients and the mixture so obtained is maintained at a temperature and for a time sufficient to ensure the desired degree of lactose hydrolysis has taken place.